# EUROPÄISCHE PATENTSCHRIFT

(11) **EP 1 177 804 B1**
(45) Veröffentlichungstag und Bekanntmachung des Hinweises auf die Patenterteilung: **14.09.2005**
(21) Anmeldenummer: 01116810.1
(22) Anmeldetag: 10.07.2001
(51) Int. Cl.: A61M 5/50

(54) **Vorgefüllte Einmalspritze und Verfahren zu ihrer Herstellung**
Pre-filled single-use syringe and process for producing it
Seringue pre-remplie à usage unique et son procédé de fabrication

(30) Priorität: 28.07.2000 DE 10036829
(43) Veröffentlichungstag der Anmeldung: 06.02.2002
(73) Patentinhaber: Schott AG, 55122 Mainz (DE)
(72) Erfinder: Heinz, Jochen, Dr., 55578 Vendersheim (DE); Spallek, Michael, Dr., 55218 Ingelheim (DE)
(74) Vertreter: Fuchs Mehler Weiss & Fritzsche

(56) Entgegenhaltungen:
- WO-A-92/10355
- GB-A- 1 150 980

## Beschreibung

Vorgefüllte Einmalspritze, mit einem Spritzenkörper, in dem ein Kolbenstopfen mittels einer Kolbenstange axial verschiebbar aufgenommen ist und der an einem Ende einer Spitzenflansch besitzt, wobei in der befüllten und mit dem Kolben versehenen Spritze mindestens eine aus verdrängtem Material des Spritzenkörpers stammende, den inneren Durchmesser des Spritzenkörpers verengende Materialanformung als Kolbenbremse gegen ein unbeabsichtigtes Herausziehen des Kolbens ausgebildet ist.

Die Erfindung betrifft ferner ein Verfahren zu ihrer Herstellung.

Für zahlreiche Applikationszwecke in der Medizin, d.h. für pharmazeutische und diagnostische Zwecke, aber auch für kosmetische Zwecke, sind vorgefüllte Einmalspritzen, auch Fertigspritzen genannt, auf dem Markt. Diese Fertigspritzen können sowohl einen Spritzenkörper aus Glas als auch aus Kunststoff aufweisen.

Fertigspritzen aus Glas sind in der DIN ISO 11040 Teil 4 beschrieben.

Eine große Anzahl verschiedenster Glas-Fertigspritzen ist in dem Aufsatz von R. D. Anand in der Zeitschrift "Pharmazeutische Industrie" **54** Nr. 1, 1992, Seite 69 - Seite 73 beschrieben.

Fertigspritzen aus Kunststoff sind aus der DE 44 38 360 A 1 oder aus der WO92/10355 bekannt.

Die Verarbeitung von Fertigspritzen wird in dem Aufsatz von E. Venten und J. Hoppert, in "Pharmazeutische Industrie" 40 Nr. 6, 1978, Seite 665 - Seite 671 sowie in "Die neue Verpackung" Band 31, 7/1978, Seiten 1062 - 1074 beschrieben.

Ein besonderes Problem bei diesen Fertigspritzen ist es, sicherzustellen, daß während der Applikation oder Vorbereitung der Fertigspritze ein unbeabsichtigtes Herausziehen des beweglichen Elastomerkolbens aus dem Spritzenkörper nicht möglich ist. Dies ist sehr wichtig, z.B. bei Spritzen, die mit Lösungsmitteln befüllt und die zum Auflösen eines pulverförmigen, insbesondere lyophilisierten Produktes dienen, das in seiner gelösten Form wieder in die Spritze gezogen werden muß, bevor es in der üblichen Weise appliziert wird.

Zur Verhinderung dieses Auszug-Effektes schlägt die DIN-ISO 11040-4 ein nach innen in den Spritzenkörper überstehenden Glasrand vor, der jedoch fertigungsbedingt nur mit äußerst geringen Abmessungen ausgeführt werden kann, so daß es ohne weiteres möglich ist, den Elastomerkolben zum hinteren Ende einer Glasfertigspritze hinauszuziehen.

Um das unbeabsichtigte Herausziehen sicher zu verhindern, wird in der DE 43 31 137 A 1 ein zusätzliches Kunststoffteil näher erläutert, das über die Fingerauflage des Spritzenkörpers geschoben wird und als mechanische Sperre gegen das Herausziehen des Elastomerkolbens wirkt.

Diese bekannte Kolben- oder Stopfenbremse wird von einem den Spritzenkörper umgreifenden, an diesem nach seiner Befüllung lösbar angebrachten Rastteil gebildet, das einen in den lichten Innenquerschnitt des Spritzenkörpers hinein vorstehenden Vorsprung aufweist, der einen Anschlag für die dem Spritzenkopf abgewandte Seite des Elastomerstopfens bildet.

Ein anderer Lösungsweg für das Anbringen einer Kolbenbremse ist durch die US 5, 688,252 bekannt geworden. Diese Schrift zeigt eine speziell gestaltete Fingerauflage, die als separates Teil nach dem Befüllen der Spritze und dem Einführen des Stopfens auf das offene Ende des Spritzenkörpers geschoben wird und die einen nach innen gerichteten, ringförmigen Überstand aufweist, der einen Anschlag für das unbeabsichtigte Herausziehen des Stopfens bzw. des Kolbens bildet.
Eine ähnliche Kolbenbremse zeigt die WO 92/08507, die eine Fertigspritze beschreibt, bei der eine separate Fingerauflage auf das Ende eines Spritzenzylinders aufgesetzt wird, der einen entsprechenden Überstand nach innen aufweist.
Die US 4,543,093 schließlich beschreibt eine Kolbenbremse für eine Fertigspritze, bei der am offenen Ende des Spritzenkörpers in seinem Innern einstückig ein umlaufender Ringwulst angeformt ist, der einem unbeabsichtigten Herausziehen des Stopfens bzw. Kolbens unter Bildung eines Anschlages entgegenwirkt.

Der vorbeschriebene Stand der Technik weist gravierende Nachteile auf, müssen doch entweder zusätzliche Teile am Spritzenkörper aufwendig montiert werden, oder es ist eine Verringerung des Innenquerschnitts des Spritzenkörpers notwendig, was die übliche Befüllung mittels eines Füllrohres behindert und Füllrohre mit kleinerem Durchmesser notwendig macht, was wiederum die Ausflußgeschwindigkeit des Füllmediums und damit die Ausbringleistung der Füllmaschine entscheidend beeinflußt.

Ferner fehlt bei allen Systemen die an sich erwünschte Einführschräge für das Füllrohr und das Setzrohr für den Elastomerkolben, denn üblicherweise werden Fertigspritzen durch ihr hinteres Ende befüllt und der Elastomerkolben wird in vorkomprimierter Form durch das Setzrohr in den Spritzenkörper eingebracht, indem das Setzrohr ins Innere des Spritzenkörpers positioniert wird und der im Setzrohr komprimierte Elastomerkolben durch das Setzrohr ausgeschoben wird und das Setzrohr direkt in seiner Position im Spritzenkörper verläßt. Da Elastomerkolben nur begrenzt komprimierbar sind, sollte wiederum das Setzrohr einen möglichst großen Durchmesser aufweisen, der gerade noch ins Innere des Spritzenkörpers paßt. Innere Überstände stehen daher dieser Forderung diametral gegenüber.

Ferner können sich beim Einführen von Füllrohr bzw. Stopfensetzrohr durch Berührungen der nach innen gerichteten Überstände am Spritzenkörper Partikel bilden, die wiederum mit Nachteil direkt in die Spritze eingebracht werden können.

Ebenfalls störend ist bei beiden Lösungsvarianten, den Weg mit der separaten Fingerauflage und dem inneren Überstand, der Effekt der schwierigen Einführung der Kolbenstange.

Schließlich ist bei beiden Lösungsvarianten ein Originalitätsverschluß nicht gegeben, d.h. die Kolbenbremsen können auch durch ungeschultes Personal leicht aufgehoben und der Inhalt manipuliert werden, ohne daß dies äußerlich an der Spritze sichtbar wäre.

Der Erfindung liegt die Aufgabe zugrunde, die eingangs bezeichnete vorgefüllte Einmalspritze mit einem Spritzenkörper, in dem ein Kolben aufgenommen ist, der mittels einer Kolbenstange axial verschiebbar ist und der eine Kolbenbremse gegen unbeabsichtigtes Herausziehen des Kolbens besitzt, so auszubilden, daß auf konstruktiv einfache Weise das Herausziehen des Kolbens sicher verhindert wird, und die vorgenannten Einschränkungen beim Befüllen der Spritze und dem Setzen des Kolbens nicht gegeben sind.

Ferner soll bei eingebrachter Kolbenstange ein Originalitätsverschluß gewährleistet sein, d.h. Manipulationen am hinteren Spritzenende sollen nur zerstörend möglich sein.

Die Lösung dieser Aufgabe gelingt erfindungsgemäß dadurch, daß die Materialanformung im Bereich der Dicke des Spritzenflansches des Spritzenkörpers ausgebildet ist.

Beim Befüllen der Spritze und dem Setzen des Kolbens steht somit der gesamte freie Querschnitt des Spritzenkörpers für das Einführen des Füll- und des Setzrohres in den Spritzenkörper zur Verfügung und erst nach der Befüllung und dem Setzen des Kolbens wird aus dem Material der Wandung des Spritzenkörpers oder der Fingerauflage die als Kolbenbremse wirkende, den freien Querschnitt verengende Materialanhäufung angeformt, die auch ein zerstörungsfreies Herausschrauben der typischerweise kreuzförmig ausgebildeten Kolbenstange aus dem Kolbenstopfen verhindert.

Um eine sichere Funktion der Kolbenbremse und des Originalitätsverschlusses zu gewährleisten, ist gemäß einer Weiterbildung der Erfindung die Einmalspritze so ausgebildet, daß peripher mehrere punktuelle Materialanhäufungen angeformt sind.

Zweckmäßig sind dabei an vier gegenüberliegenden Stellen Materialanhäufungen angeformt.

Grundsätzlich kann auch eine ringförmig umlaufende Materialanhäufung angeformt sein.

Die Herstellung der erfindungsgemäßen vorgefüllten Einmalspritze erfolgt unter verfahrensmäßiger Lösung der vorgenannten Aufgabe erfindungsgemäß mit den Schritten:
- Bereitstellen des befüllten und mit dem Kolben versehene Spritzenkörpers, und
- stellenweises Erwärmen des Spritzenflansches des vorgefüllten Spritzenkörpers und Verformen der erwärmten Stellen unter Verdrängen von Material des Spritzenflansches zu einer in den freien Querschnitt des befüllten Spritzenkörpers ragenden Materialanformung.

Gemäß einer Ausgestaltung wird dabei das Erwärmen und Verformen mittels einer beheizten Zange durchgeführt. Alternativ dazu kann das Erwärmen und Vorformen auch durch eine äußere Strahlungsquelle in Verbindung mit einem Druckwerkzeug durchgeführt werden.

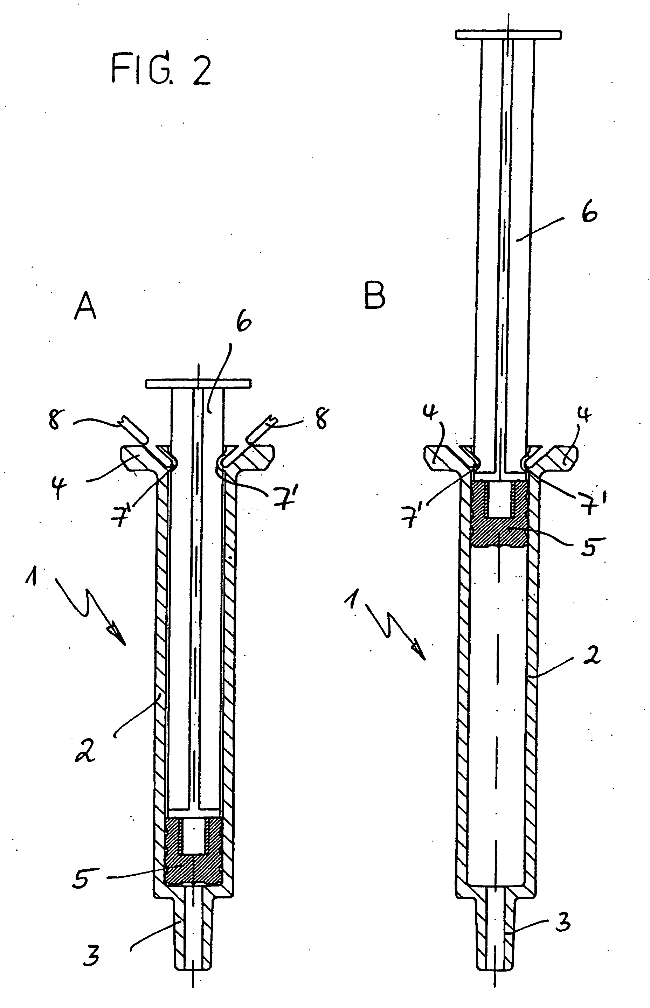

Anhand der Beschreibung von zwei in den Zeichnungen dargestellten Ausführungsformen der Erfindung wird diese näher erläutert. Nur Figur 2 zeigt die Erfindung.

Es zeigen:
- Fig. 1: in zwei Figurenteilen A, B in einer Längsschnitt-Darstellung eine vorgefüllte Einmalspritze mit einer ersten Ausführungsform einer Kolbenbremse, mit eingefahrenem Kolben im Figurenteil A und ausgezogenem Kolben im Figurenteil B, und
- Fig. 2: ebenfalls in zwei Figurenteilen A, B in einer Längsschnitt-Darstellung eine vorgefüllte Einmalspritze mit einer zweiten Ausführungsform einer Kolbenbremse, mit eingefahrenem Kolben im Figurenteil A und ausgezogenem Kolben im Figurenteil B.

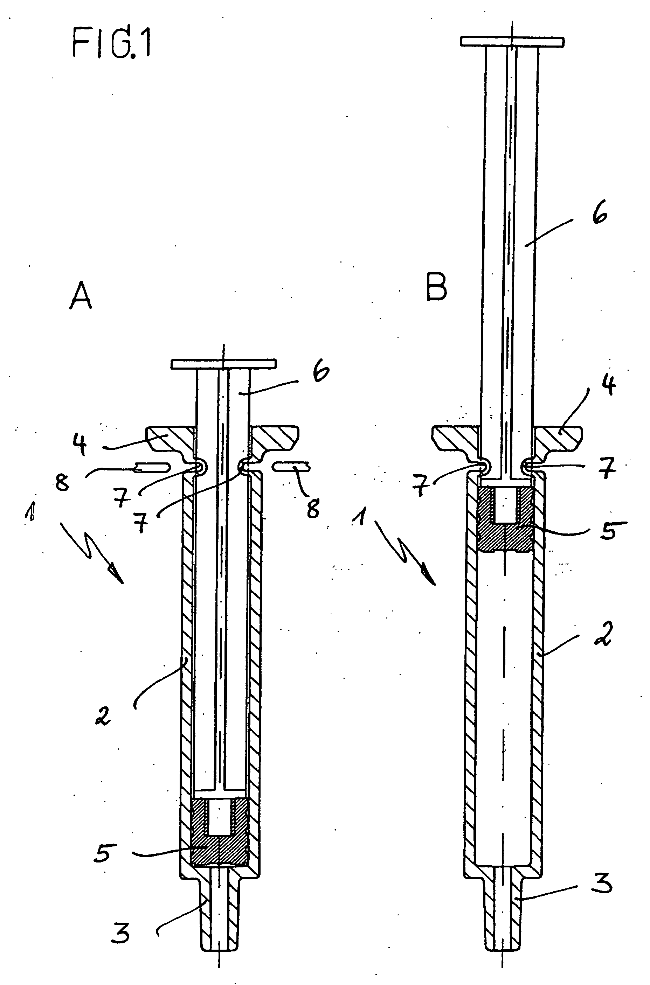

Die Fig. 1 zeigt eine erste Ausführungsform der erfindungsgemäßen Ausbildung einer Kolbenbremse an einer vorfüllbaren Einmalspritze, und zwar im Figurenteil A im eingeschobenen Zustand des Kolbens und im Figurenteil B im Ausgangszustand der vorfüllbaren Einmalspritze. Die dargestellte vorgefüllte Einmalspritze, im folgenden als Fertigspritze bezeichnet, weist einen Spritzenkörper 1 auf, der im dargestellten Ausführungsbeispiel aus einem Spritzenzylinder 2, einem daran kopfseitig angeformten Luer-Konus 3 und einem daran am gegenüberliegenden Ende angeformten Spritzenflansch 4 besteht. Im gefüllten Zustand ist dabei der Luer-Konus durch ein sogenanntes Tip Cap (nicht dargestellt) verschlossen, das im Applizierfall abgenommen und durch einen Nadelträger ersetzt wird.

Andere Ausführungsformen des Spritzenkörpers sind denkbar, z.B. kann dieser anstelle eines kreisrunden Querschnittes auch einen mehreckigen Querschnitt besitzen, einen Spritzenkopf mit integrierter Nadel, die durch eine Nadelkappe abgedeckt ist, aufweisen, oder eine separate, aufsteckbare Fingerauflage besitzen.

In dem Spritzenzylinder 2 ist ein stopfenförmiger Kolben 5 aus einem elastomeren Material eingeschoben, in den eine Kolbenstange 6 einsteckbar ist, mittels der der Elastomer-Kolben 5 in dem Spritzenzylinder 2 axial verschiebbar ist. Diese Verschiebbarkeit wird dabei durch eine auf der Innenwandung des Spritzenzylinders 2 aufgebrachte (nicht dargestellte) Silikon-Gleitschicht unterstützt.

Im Ausgangszustand, d.h. vor dem Befüllen der Spritze und dem Setzen des Elastomer-Kolbens 5 mit bekannten Methoden und Einrichtungen, weist der Spritzenzylinder 2 durchgehend eine gleiche Wandstärke ohne innere Überstände oder dergleichen auf. Das Befüllen und Setzen des Kolbens 5 kann daher mit maximalen Durchmessern des Füll- und Setzrohres erfolgen. Erst nach dem Befüllen des Spritzenzylinders 2 und Setzen des Elastomer-Kolbens 5 wird die erfindungsgemäße Kolbensperre angebracht, indem der Spritzenzylinder 2 punktförmig von außen erwärmt und eingedrückt wird und somit punktförmige, weit ins Innere des Spritzenkörpers 1 ragende Materialansammlungen 7 entstehen, die den Elastomer-Kolben 5 auch mit eingeschobener Kolbenstange 6 sicher blockieren.

Dieses Erwärmen und Eindrücken von außen erfolgt mittels geeigneter Werkzeuge 8, die in Fig. 1 A symbolisch angedeutet sind.

Das Verfahren eignet sich vorzugsweise für Spritzenkörper 2 aus Kunststoff. Beispielsweise können als Werkzeuge 8 beheizte Zangen zur Erhitzung und Verformung eingesetzt werden oder es kann auch ein punktförmiges Aufheizen über Strahlungsquellen mit anschließendem mechanischen Umformen erfolgen.

In Fig. 1 sind radiale Einschnürungen 7 der Wandung des Spritzenkörpers 2 an dessen austrittsseitigem Ende dargestellt, die die Kolbenbremse bilden.

Bei der zweiten, in Fig. 2 dargestellten Ausführungsform der erfindungsgemäßen Ausbildung der Kolbenbremse an einer Fertigspritze werden die in das Innere des Spritzenkörpers 1 ragenden Materialansammlungen 7' unter einem Winkel zur Spritzenachse im Spritzenflansch 4 ausgeformt, mit Methoden und einer Vorrichtung 8, wie sie im Zusammenhang mit der Fig. 1 bereits beschrieben worden sind.

Im übrigen ist der Aufbau der Fertigspritze nach Fig. 2 identisch zu derjenigen nach Fig. 1, was auch durch die Gleichheit der Bezugsziffern zum Ausdruck gebracht ist. Auch die im Zusammenhang mit der Fig. 1 beschriebenen Varianten sind bei der Ausführungsform nach Fig. 2 denkbar.

## Patentansprüche

1. Vorgefüllte Einmalspritze, mit einem Spritzenkörper (1), in dem ein Kolbenstopfen (5) mittels einer Kolbenstange (6) axial verschiebbar aufgenommen ist und der an einem Ende einer Spitzenflansch (4) besitzt, wobei in der befüllten und mit dem Kolben (5) versehenen Spritze mindestens eine aus verdrängtem Material des Spritzenkörpers (1) stammende, den inneren Durchmesser des Spritzenkörpers (1) verengende Materialanformung (7') als Kolbenbremse gegen ein unbeabsichtigtes Herausziehen des Kolbens (5) ausgebildet ist, **dadurch gekennzeichnet, dass** die Materialanformung (7') im Bereich der Dicke des Spritzenflansches (4) des Spritzenkörpers (1) ausgebildet ist.

2. Vorgefüllte Einmalspritze nach Anspruch 1, **dadurch gekennzeichnet, dass** peripher mehrere punktuelle Materialanformungen (7') vorgesehen sind.

3. Vorgefüllte Einmalspritze nach Anspruch 1, **dadurch gekennzeichnet, dass** eine ringförmig umlaufende Materialanformung vorgesehen ist.

4. Vorgefüllte Einmalspritze nach einem der Ansprüche 1 bis 3, **dadurch gekennzeichnet, dass** der Spritzenkörper (1) aus Kunststoff besteht.

5. Vorgefüllte Einmalspritze nach einem der Ansprüche 1 bis 4, **dadurch gekennzeichnet, dass** der Kolben (5) aus einem elastomeren Material besteht.

6. Verfahren zum Herstellen der vorgefüllten Einmalspritze nach einem der Ansprüche 1 bis 5, mit den Schritten:
- Bereitstellen des befüllten und mit dem Kolben versehenen Spritzenkörpers, und
- stellenweises Erwärmen des Spritzenflansches des vorgefüllten Spritzenkörpers und Verformen der erwärmten Stellen unter Verdrängen von Material des Spritzenflansches zu einer in den freien Querschnitt des befüllten Spritzenkörpers ragenden Materialanformung.

7. Verfahren nach Anspruch 6 zum Herstellen der vorgefüllten Einmalspritze nach Anspruch 2, bei dem das Erwärmen und das Verformen mittels einer beheizten Zange durchgeführt wird.

8. Verfahren nach Anspruch 6, bei dem das Erwärmen durch eine äußere Strahlungsquelle und das Verformen durch ein Druckwerkzeug durchgeführt wird.

## Claims

1. Pre-filled single-use syringe, with a syringe body (1) in which a plunger stopper (5) is received in such a way that it can be moved axially by means of a plunger rod (6), and with a syringe flange (4) at one end, where, when the syringe is filled and is provided with the plunger (5), at least one material accumulation (7'), originating from displaced material of the syringe body (1) and narrowing the internal diameter of the syringe body (1), is formed as a plunger brake against inadvertent withdrawal of the plunger (5), **characterized in that** the material accumulation (7') is formed in the area of the thickness of the syringe flange (4) of the syringe body (1).

2. Pre-filled single-use syringe according to Claim 1, **characterized in that** a plurality of material accumulations (7') are provided at points about the periphery.

3. Pre-filled single-use syringe according to Claim 1, **characterized in that** one annular material accumulation is provided about the circumference.

4. Pre-filled single-use syringe according to one of Claims 1 to 3, **characterized in that** the syringe body (1) is made of plastic.

5. Pre-filled single-use syringe according to one of Claims 1 to 4, **characterized in that** the plunger (5) is made of an elastomer material.

6. Process for producing the pre-filled single-use syringe according to one of Claims 1 to 5, comprising the following steps:
- making ready the filled syringe body provided with the plunger,
- heating the syringe flange of the pre-filled syringe body in some areas and shaping the heated areas in order to displace material of the syringe flange and so form a material accumulation which protrudes into the free cross section of the filled syringe body.

7. Process according to Claim 6 for producing the pre-filled single-use syringe according to Claim 2, in which the heating and shaping are carried out by means of heated tongs.

8. Process according to Claim 6, in which the heating is carried out by an external radiation source, and the shaping by a pressure tool.

## Revendications

1. Seringue pré-remplie à usage unique avec un corps (1) de seringue destiné à recevoir un bouchon (5) de piston pouvant être déplacé axialement au moyen d'une tige (6) de piston et qui présente en une extrémité une bride (4) de seringue, au moins une partie façonnée (7') de matériau provenant du matériau repoussé du corps (1) de seringue, rétrécissant le diamètre interne du corps (1) de seringue dans la seringue remplie et pourvue du piston (5) étant réalisée comme frein de piston contre un retrait non intentionnel du piston (5), **caractérisée en ce que** la partie façonnée (7') de matériau est réalisée au niveau de l'épaisseur de la bride (4) du corps (1) de la seringue.

2. Seringue pré-remplie à usage unique selon la revendication 1, **caractérisée en ce qu'**on a prévu sur la périphérie, plusieurs parties façonnées (7') de matériau ponctuelles.

3. Seringue pré-remplie à usage unique selon la revendication 1, **caractérisée en ce qu'**on a prévu une partie façonnée de matériau périphérique annulaire.

4. Seringue pré-remplie à usage unique selon l'une quelconque des revendications 1 à 3, **caractérisée en ce que** le corps (1) de seringue est en matériau synthétique.

5. Seringue pré-remplie à usage unique selon l'une quelconque des revendications 1 à 4, **caractérisée en ce que** le piston (5) est en un matériau élastomère.

6. Procédé de fabrication de la seringue pré-remplie à usage unique selon l'une quelconque des revendications 1 à 5, comprenant les étapes suivantes :
- préparation du corps de seringue rempli, pourvu du piston et
- chauffage par endroits de la bride de la seringue du corps de seringue pré-rempli et façonnage des endroits réchauffés en repoussant le matériau de la bride de la seringue en une partie façonnée s'avançant dans la section libre du corps de seringue rempli.

7. Procédé selon la revendication 6 pour la fabrication de la seringue pré-remplie à usage unique selon la revendication 2, dans lequel le chauffage et le façonnage sont réalisés au moyen d'une pince chauffée.

8. Procédé selon la revendication 6, dans lequel le chauffage est réalisé par une source d'irradiation externe et le façonnage par un outil de pression.
